# EUROPEAN PATENT SPECIFICATION

(11) **EP 0 939 752 B1**
(45) Date of publication and mention of the grant of the patent: **10.08.2005**
(21) Application number: 96944385.2
(22) Date of filing: 17.12.1996
(51) Int. Cl.: C07C 51/58, C07C 59/68, C07C 41/03

(54) **MANUFACTURE OF FLUORINATED COMPOUNDS**
HERSTELLUNG FLUORINIERTER VERBINDUNGEN
PRODUCTION DE COMPOSES FLUORES

(30) Priority: 22.12.1995 US 9128 P
(43) Date of publication of application: 08.09.1999
(73) Proprietor: E.I. DU PONT DE NEMOURS AND COMPANY, Wilmington, Delaware 19898 (US)
(72) Inventor: FARNHAM, William, Brown, Hockessin, DE 19707 (US); RESNICK, Paul, Raphael, Cary, NC 27511-7802 (US); SUBRAMANYAM, Vinayakam, Boothwyn, PA 19061-1616 (US)
(74) Representative: Matthews, Derek Peter
(86) International application number: PCT/US1996/019972
(87) International publication number: WO 1997/023440

(56) References cited:
- EP-A- 0 281 959
- GB-A- 1 185 565
- US-A- 3 694 499
- RESEARCH DISCLOSURE, vol. 112, August 1973, HAVANT GB, pages 10-11, XP002028363 E. I. DU PONT DE NEMOURS: "Synthesis of perfluoro(5-phenoxy-2-methyl-3-oxacaproyl fluoride)"
- JOURNAL OF FLUORINE CHEMISTRY, vol. 9, no. 2, 1976, LAUSANNE CH, pages 97-112, XP000646442 J. T. HILL: "Octafluoroisobutylene epoxide derivatives"

## Description

### FIELD OF THE INVENTION

Described herein is an improved process for making pentafluorophenoxy substituted perfluoroethers by reacting a pentafluorophenoxide salt with hexafluoropropylene oxide under specified process conditions. The resulting product, which is useful as an intermediate for the preparation of a monomer, is often obtained in improved yield, and/or the use of ozone depleting solvents is greatly reduced or avoided.

### TECHNICAL BACKGROUND

Perfluorinated pentafluorophenoxy substituted vinyl ethers are useful as curesite monomers for perfluoroelastomers. These compounds can be made by the reaction of a pentafluorophenoxide salt (PFPS) with hexafluoropropylene oxide (HFPO) to yield a corresponding pentafluorophenyl substituted ether containing an acyl fluoride group. The acyl fluoride may then be converted to a vinyl ether, thereby forming the desired monomer.

In the reaction of the PFPS with HFPO, highly fluorinated (which are sometimes also chlorinated, so-called CFC compounds) solvents which can act to decrease atmospheric ozone levels have typically been used. Also a typical problem in these processes has been obtaining a high yield of any particular oligomer desired, represented by "n" in the formula C₆F₅O[CF(CF₃)CF₂O]ₙCF(CF₃)COF, wherein n is 0 or a lower integer. It would therefore be desirable to have a process for producing such compounds which eliminates the use of ozone depleting compounds, and/or more selectively produces the desired oligomer.

GB-A-1185565 discloses novel perfluorinated acyl fluorides useful in the preparation of polymerizable monomers.

US 3,694,499 discloses compounds of the structure: 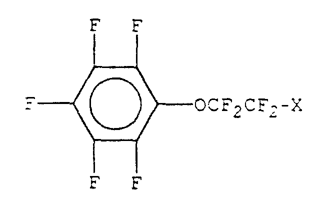 are provided wherein X is -COF, -COCl, -COOH, -COOR, -COOM, -CONR₁R₂, or -CN wherein R is an alkyl radical, M is a metal, and R₁ and R₂ are independently hydrogen or alkyl radicals. Also provided are novel compounds of the formula C₆F₅OCF₂CF₂CF₂OCF(CF₃)-X wherein X is as defined above. These novel compounds are useful in the preparation of perfluoro(3-phenoxypropyl vinyl ether) monomer, said monomer being useful in the preparation of copolymers having improved oxidative stability.

EP-A-281959 claims a process for preparing halogenated carboxylic acid fluorides by reacting a carboxylic acid fluoride with hexafluoropropene oxide in the presence of a catalyst, which comprises reacting a carboxylic acid fluoride of the formula

R-COF (III)

in which is the group CCl₃- or X-(CF₂)ₙ, X representing a hydrogen atom, a bromine atom or a fluorosulfonate radical and n denoting an integer from 1 to 6, with hexafluoropropene oxide in the presence of a catalyst system comprising an alkali metal fluoride, a carboxylic acid dinitrile and at least one ether of the formula

CH₃O-(CH₂CH₂O)₂-CH₃ (V)

in which z denotes an integer from 2 to 6.

Research Disclosure, vol. 112, August 1973, Havant pages 10-11 discloses synthesis of perfluoro(5-phenoxy-2-methyl-3-oxacaproyl fluoride), and J. Fluorine Chemistry, vol. 9, 1977, Hill pages 97-112 discloses reaction of C₆F₅O(CF₃)CFCF₂O⁻ Cs⁺ with octafluoroisobutylene oxide, OFIBO, in tetraglyme.

### SUMMARY OF THE INVENTION

This invention concerns, a first process for the production of a pentafluorophenoxy substituted perfluoroether, comprising, contacting, in a solvent mixture consisting essentially of tetraglyme and 1,2-dimethoxyethane or methylene chloride or a mixture thereof, and at a temperature of about -60°C to about 0°C, an alkali metal salt of pentafluorophenol and hexafluoropropylene oxide to produce a compound of the formula C₆F₅OCF(CF₃)CF₂OM (I), wherein M is said alkali metal;
provided that the total molar amount of said hexafluoropropylene oxide added to the process is about 1.0 to about 1.25 times the molar amount of said alkali metal salt of pentafluorophenol present in said process.

This also concerns a second process for the production of a pentafluorophenoxy substituted perfluoroether, comprising, contacting, at a temperature of about +10°C to about +80°C, in a solvent mixture consisting essentially of adiponitrile and tetraglyme, a compound of the formula C₆F₅OCF(CF₃)COF (III), with a catalytic amount of potassium fluoride and hexafluoropropylene oxide to produce a compound of the formula C₆F₅OCF(CF₃)CF₂OCF(CF₃)COF (II).

Also disclosed herein is a third process for the production of a pentafluorophenoxy substituted perfluoroether, comprising,
(a) contacting, in a solvent mixture consisting essentially of tetraglyme and 1,2-dimethoxyethane or methylene chloride or a mixture thereof, and at a temperature of about -60°C to about 0°C, an alkali metal salt of pentafluorophenol and hexafluoropropylene oxide to produce a compound of the formula C₆F₅OCF(CF₃)CF₂OM (I), wherein M is said alkali metal;
   provided that the total molar amount of said hexafluoropropylene oxide added to the process is no greater than 1.25 time the molar amount of said alkali metal salt of pentafluorophenol present in said process to produce (I); and
(b) heating (I) to produce a compound of the formula C₆F₅OCF(CF₃)COF (III);
(c) contacting, at a temperature of about +10°C to about +80°C, in a solvent mixture consisting essentially of adiponitrile and tetraglyme, (III) with a catalytically effective amount of potassium fluoride and hexafluoropropylene oxide to produce a compound of the formula C₆F₅OCF(CF₃)CF₂OCF(CF₃)COF (II).

Also described herein is a fourth process for the production of a pentafluorophenoxy substituted perfluoroether, comprising:
(a) contacting, in a solvent mixture consisting essentially of tetraglyme and 1,2-dimethoxyethane or methylene chloride or a mixture thereof, and at a temperature of about -60°C to about 0°C, an alkali metal salt of pentafluorophenol and hexafluoropropylene oxide to produce a compound of the formula C₆F₅OCF(CF₃)CF₂OM (I), wherein M is said alkali metal;
   provided that the total molar amount of said hexafluoropropylene oxide added to the process is no greater than 1.25 time the molar amount of said alkali metal salt of pentafluorophenol present in said process to produce (I):
(b) adding a perfluorinated compound which is a liquid under the process conditions; and
(c) then heating to about 25°C to about 50°C while adding approximately at least about one equivalent of HFPO to produce a compound of the formula C₆F₅O[CF(CF₃)CF₂O]ₙCF(CF₃)COF (V), wherein n is an integer of 1 to 5.

### DETAILS OF THE INVENTION

In the first process described herein one of the ingredients is a PFPS, which is also sometimes referred to an alkali metal pentafluorophenoxide. In a preferred PFPS the alkali metal is sodium or potassium, more preferably potassium. Since different alkali metal ions which may be present may promote the desired reaction or other side reactions at different rates, it may be necessary to adjust the first process conditions particularly in regards to temperature and the exact solvent mixture used (see below) to obtain the desired product. Some of these conditions are illustrated in the Examples, and others may be readily determined by simple experimentation.

In the first process a solvent mixture is employed. This mixture must be a liquid at the process temperatures, must dissolve at least some of the PFPS (preferably at least about 25 g/L at the process temperature, more preferably at least about 50 g/L, and especially preferably 100 g/L), and should not significantly react with HFPO or the PFPS (i.e. should be chemically inert). The ratio of the two (or more) compounds in the solvent mixture may be adjusted by simple experimentation to maximize the yield of the desired product. Useful solvents for the mixture are tetraglyme combined with one or more of a second compound, i.e. 1,2-dimethoxyethane and/or methylene chloride. It is preferred that the tetraglyme is about 80 to about 95 volume percent of the solvent mixture.

In the first process it is preferred that the temperature is about -30°C to about -15°C. It is also preferred that the molar ratio of HFPO to PFPS is about 1.0 to about 1.15. A preferred method of running the process is to add the PFPS to the solvent mixture, cool to the desired temperature and then add the HFPO at such at rate so the temperature can be controlled. Other methods may also be used to run the reaction, but care should be taken to be able to control the temperature during the exothermic reaction.

The initial product of the first process is an alkali metal salt, preferably a potassium salt of formula (I). The alkali metal salt may be converted to the acyl fluoride (which is a starting material for the second process herein) by heating, typically under reduced pressure, at a temperature of about 20°C to about 150°C, preferably about 25°C to about 100°C, for a period of time sufficient to effect the transformation. This time period is typically 1 hr. to 1 day, depending mostly on the scale of the reaction (including time needed to remove the second compound of the solvent mixture). The acyl fluoride (III) may be isolated by distillation. Distillation is easier if the compounds employed in the solvent mixture have boiling points substantially different from the desired product.

In the second process herein a starting material is the acyl fluoride (III) which is produced by heating the (intermediate) product of the first process. A catalytically effective amount of a fluoride ion source is also present, and preferably the fluoride ion source is potassium fluoride. A useful amount of potassium fluoride is about 2 to 10 mole percent of the amount of acyl fluoride charged.

A solvent mixture is also present in the second process. This mixture consists essentially of adiponitrile [NC(CH₂)₄CN] and tetraglyme [CH₃O(CH₂CH₂O)₄CH₃]. It is preferred that the adiponitrile is about 75 to about 95 volume percent of the solvent mixture.

In the second process it is preferred that the temperature is about 30°C to about 50°C. It is also preferred that the molar ratio of HFPO to the initial acyl fluoride is about 1.0 to about 1.2. A preferred method of running the process is to add the initial acyl fluoride and catalyst to the solvent mixture (in which it is at least partially soluble), adjusting to the desired temperature and then add the HFPO at such at rate as to control the temperature. Other methods may also be used to run the reaction, but care should be taken to be able to control the temperature during the exothermic reaction.

As will be recognized, the third process herein is a combination the first and second processes, and all of the discussions and preferred items for the first and second processes, and the heating of (I) to produce (III), apply as well to their respective parts of the third process.

Alternatively, a fourth process may be run to produce the desired final product (the above mentioned product of the second process). After the first process has been carried out, a completely perfluorinated compound which is a liquid under the process conditions is added, typically about one-half to an equal volume of the perfluorinated liquid to the volume of the initial solvent mixture, and then the process ingredients are heated to about 25°C to about 50°C while adding approximately at least one equivalent of HFPO. The larger the amount of HFPO added, the higher "n" in (V) will be, on average. Useful perfluorinated liquids include perfluoro(n-butyltetrahydrofuran), hexafluoropmpylene cyclic dimer, and other relatively low boiling perfluorinated (poly)ethers. The desired product may then be isolated by distillation.

In all of the processes herein it is preferred to exclude moisture and oxygen, especially moisture, since they may lead to undesired side reactions and reduce the yield of the desired product. Therefore reaction ingredients should preferably be dry. The processes should be agitated to ensure reasonably good mixing of the ingredients.

In the Examples, the following abbreviations are used:
FC-75 - perfluoro(n-butyltetrahydrofuran), available from the 3M Company, Minneapolis, MN, U.S.A.
GC - gas chromatography
glyme - 1,2-dimethoxyethane
(HFPO)₂- ester - CF₃CF₂CF₂OCF(CF₃)C(O)OC₆F₅

In the Examples the products are sometimes referred to as x/y adducts, wherein x and y are integers. X refers to the number of "moles" of pentafluorophenoxy groups in the molecule, and y refers to the number of HFPO units in the molecule. "Ester" refers to the pentafluorophenyl ester of the corresponding acyl fluoride.

### EXAMPLE 1

A ca. 1000 mL resin kettle was fitted with overhead stirrer, thermocouple, combination HFPO addition port and dry ice condenser attached to a nitrogen line, and solid addition port (jointed glass tube which extended into the reaction vessel) was dried and then charged with dry tetraglyme (200 mL), glyme (22 mL), and C₆F₅OK (40.04 g, 180 mmol). The mixture was then cooled to -35°C. HFPO (33.0 g, 199 mmol) was added using a mass flow controller and the temperature of the reaction mixture was maintained at -31°C to -35°C. After 20 min at -35°C, the mixture was warmed to -10°C in stages (ca. 0.5 h), and was sampled for GC analysis which showed functional equivalents of (area%): 1/1 COF = 89.5%, 1/2 COF = 7.6 %, 1/1 C₆F₅ ester = ester = 2.9%, 1/2 C₆F₅ ester = not detected.

### COMPARATIVE EXAMPLE

FC-75 (108 g) was added to the reactor of Example 1, and the mixture was warmed to 10°C. Another 33 g HFPO (199 mmol) was added to the stirred mixture while the temperature was maintained at ca. 10°C. When addition was complete, the mixture was warmed to room temperature. GC analysis of the top layer and bottom layer were carried out separately. Top layer showed functional equivalents of (relative area%): 1/1 COF = 15%, 1/2 COF = 66%, 1/3 COF = 18%, 1/4 COF = 1%, 1/1 C₆F₅ ester = <1%. Using tetraglyme signal as a standard, it appeared that ca. 78% of fluorocartion material was still present in the hydrocarbon phase. The bottom layer showed functional equivalents of (relative area%): 1/1 COF = 1%, 1/2 COF = 39%, 1/3 COF = 43%, 1/4 COF = 15%, 1/5 COF = 2%. To determine the overall composition, the lower-boiling materials were collected at 13 Pa, and the distillation was continued for a short time after the head temperature had reached that of tetraglyme. GC analysis of the resulting bottom layer showed (relative area %): 1/1 COF = 12%, 1/2 COF = 61%, 1/3 COF = 23%, 1/4 COF = 4%. Only a small amount of 1/1 and 1/2 adducts were present in the top layer, and this was discarded along with the top layer before proceeding with final distillation. This provided 44.5 g of 1/2 adduct.

The selectivity values obtained from the crude reaction mixture were about the same as the control reaction, meaning that FC-75 has little effect on distributions at this temperature.

### EXAMPLE 2

A ca. 1000 mL resin kettle was fitted with overhead stirrer, thermocouple, combination HFPO addition port and dry ice condenser attached to a nitrogen line, and solid addition port (jointed glass tube which extended into the reaction vessel) was dried and then charged with dry tetraglyme (225 mL), glyme (25 mL), and C₆F₅OK (40.04 g, 180 mmol). The mixture was then cooled to -35°C. HFPO (33.0 g, 199 mmol) was added using a mass flow controller and the temperature of the reaction mixture was maintained at -31°C to -35°C. After 20 min at -35°C, the mixture was warmed to -10°C in stages (ca. 0.6 h), and was sampled for GC analysis which showed functional equivalents of (area %): 1/1 COF = 89.7%, 1/2 COF = 7.5%, 1/1 C₆F₅ ester = 2.8%, 1/2 C₆F₅ ester = not detected.

FC-75 (100 mL) was added to the reactor, and the mixture was warmed to 35°C. Another 33 g HFPO (199 mmol) was added to the stirred mixture while the temperature was maintained at ca. 35°C. When addition was complete, the mixture was stirred for 40 min., then allowed to cool to room temperature. GC analysis of the top layer and bottom layer were carried out separately. The entire volatile portion was then collected up to the bp of tetraglyme. Cooling the resulting transferred material at 0°C gave two layers, and the small top layer contained only a very small amount of product. The bottom layer showed (relative area %): 1/1 COF = 6%, 1/2 COF = 69%, 1/3 COF = 24%, 1/4 COF = 2%. Only a small amount of 1/1 and 1/2 adducts were present in the top layer, and these were discarded along with the top layer before proceeding with final distillation.

This gave a distilled yield of 1/1 adduct of 4.6 g and 1/2 adduct of 56.0 g, equivalent to an overall yield of 126.8 mmol/180 mmol = 70.4%.

### EXAMPLE 3

A 250 mL Fisher/Porter vessel was charged with C₆F₅OCF(CF₃)COF (24.7 g, 74.8 mmol), 11 mL of 9/1 adiponitrile/tetraglyme and spray-dried potassium fluoride (0.35 g, 6 mmol). The pressure head was attached, and the contents were stirred for 0.5 h as a significant portion of the solid went into solution. The apparatus was attached to the HFPO line and was placed in a bath controlled at 25°C. HFPO (11.9 g, 71.7 mmol) was added over 30 min. Internal temperature increased to 29°C during the addition. Pressure returned to ca. atmospheric after 0.5 h. GC analysis of the bottom layer showed the following composition:

| component | area % |
|---|---|
| HFPO dimer+trimer | 4.3 |
| 1/1 adduct | 12.0 |
| 1/2 adduct | 78.2 |
| 1/3 adduct | 5.5 |

Another 1.78 g (10.7 mmol) of HFPO was added and the reaction mixture was stirred until pressure returned to atmospheric. Bottom layer was analyzed by GC:

| component | area % |
|---|---|
| HFPO dimer+trimer | 6.4 |
| 1/1 adduct | 5.6 |
| 1/2 adduct | 80.1 |
| 1/3 adduct | 7.9 |

Another 0.89 g (5.4 mmol) of HFPO was added and the mixture was stirred until pressure returned to atmospheric. Bottom layer was analyzed by GC:

| component | area % |
|---|---|
| HFPO dimer+trimer | 6.3 |
| 1/1 adduct | 3.0 |
| 1/2 adduct | 81.1 |
| 1/3 adduct | 9.6 |

Contents of the reactor were transferred by cannula to a flask and the bottom layer was separated and distilled using a short spinning band column @ 1.9 kPa to give a total of 27.6 g 1/2 adduct (74% based upon 1/1 adduct).

### COMPARATIVE EXAMPLE

A ca. 1000 mL resin kettle was fitted with overhead stirrer, thermocouple, combination HFPO addition port and dry ice condenser attached to a nitrogen line, and solid addition port (jointed glass tube which extended into the reaction vessel) was dried and then charged with dry glyme (200 mL) and cooled to -50°C. The HFPO addition tube extended into the reactor close to the solvent level. The solid addition tube was aimed to discharge toward the center of the reactor. The stirrer was a glass disk with attached blades. HFPO (16.5 g, 99 mmol) was added using a mass flow controller and the temperature of the reaction mixture was then lowered to -78°C. Then another 16.5 g of HFPO was added at ca. 0.5 g/min while the C₆F₅OK (40.15 g, 180 mmol) was simultaneously added over a period of ca. 45 min using a section of Gooch tubing. After stirring @ -78°C for 1 h. the mixture was warmed to -60°C. The solution was sampled for GC analysis which showed functional equivalents of (area%): 1/1 COF = 76%, 1/2 COF = 11%. 1/1 C₆F₅ ester = 10%, 1/2 C₆F₅ ester = 3%.

Reactor contents were transferred to a single neck flask for distillation using a cannula, and the volatiles were then removed under vacuum. The solid remaining in the pot was coated with product liquid, so the flask was heated in order to drive over as much product as possible. Distillation under reduced pressure afforded 41.52 g of 1/1 adduct and 4.61 g of 1/2 adduct, equivalent to a combined molar yield of 75%.

### COMPARATIVE EXAMPLE

A 250 mL 3-neck rbf fitted with overhead stirrer, thermocouple, and combination HFPO addition port and dry ice condenser attached to a nitrogen line was charged with potassium pentafluorophenoxide (8.03 g, 36 mmol), dry glyme (50 mL) and FC-75 (35 mL). The mixture was cooled to ca -78°C, and a substantial amount of the solid precipitated and clung to the flask walls, although stirring was still functional. HFPO (6.0 g, 36 mmol) was added (3-4 min), and solid disappeared. After stirring @ -78°C for 20 min, the mixture was warmed in stages to 0°C. The top layer was sampled for GC analysis which showed (area%): 1/1 COF = 90%,1/2 COF = 2%, 1/1 C₆F₅ ester = 8%.

The mixture was then warmed to 25°C and treated with another 6.00 g HFPO (addition over ca. 15-20 min on account of slower uptake). The mixture was stirred overnight. GC analysis of the lower layer showed the following composition:

| Distribution of acid fluorides |
|---|
| 1/1 COF = 27% |
| 1/2 COF = 67% |
| 1/3 COF = 6% |

Total area fraction of esters was about 5%.

Since conversion of the 1/1 adduct was judged to be lower than desired, another 3.0 g HFPO was added, again at ca. 24°C. GC then showed distribution of adducts as 15/73/12. To obtain final, overall distribution of adducts, the volatiles were vacuum transferred and solvent was then distilled to afford a single layer which was GC analyzed and showed a 24%/69%/6% distribution of adducts.

### EXAMPLE 4

A 3-liter multiport round-bottomed flask set up in a cooling bath was equipped with an air-driven overhead stirrer, a thermal well, a dry ice condenser and a gas inlet tube [reaching the lower half of the flask] connected by a Y-tube to nitrogen line and a HFPO cylinder. The flask was charged with 222 grams [1 mole] of potassium pentafluorophenoxide, 1200 ml of tetraglyme and 120 ml of dichloromethane. It was stirred vigorously and a nitrogen atmosphere was established.

The condenser was filled with acetone and dry ice. The bath was filled with acetone and dry ice added in portions until the bath temperature read -40°C where it was maintained during the HFPO addition. The contents of the flask were cooled to -32°C and HFPO was fed in at a steady rate of about 6 grams per minute and the internal temperature was maintained in the range of -27°C to -32°C. If the temperature rose above -27°C rate of addition of HFPO was reduced. A gentle reflux of HFPO was seen at the dry ice condenser. When 183 grams [1.10 moles; 10% excess] of HFPO had been added, the HFPO cylinder was closed and conditions of reaction maintained for an additional 30 minutes. The cooling bath was drained and the reaction mixture was allowed to warm up to +10°C during 3 hours while maintaining stirring. A sample of the reaction was analyzed by GC and found to be a mixture of 82% of 1:1-adduct, 8% of 2:1-adduct and 6% of ester of the acid fluoride and the phenoxide. Other impurities amounted to 4%.

The reaction was transferred to an evaporating flask and the low boiling dichloromethane was stripped in a rotary evaporator at 100 mm Hg and a bath temperature of 40-45°C. The contents were then transferred to a 2-liter distilling still equipped with a 30" x 1" column packed with Pro-pak® 316 stainless steel (0.24 x 0.24") packing [Aldrich Chemical Co.]. Distillation was carried at 35 mm Hg. After an initial collection of low boilers in the temperature range of 25° to 55°C the main bulk of the product steadily distilled in the temperature range of 55° to 68°C. It was collected and weighed. The weight was 238 grams [72% yield based on C6F5-OK] and found to be >97% 1:1-adduct. Further distillation yielded 15 grams of a mixture of 1:1 [42%] and 2:1-adduct [57%].

### EXAMPLE 5

Conditions were identical to Example 4. The only variation was the ratio of solvents. In this experiment the solvent was a mixture of 1000 ml of tetraglyme and 300 ml of dichloromethane. The GC-analysis of the product before distillation showed 85% of 1:1-adduct, 7% of 2:1-adduct and the rest ester and other unidentified impurities. Distilled yield was 248 grams [75% yield based on C6F5-OK] and a purity of 96%.

### EXAMPLE 6

Quantities were identical to those in Example 4. The reaction was carried out in a temperature range of -27°C to -21°C. Yield was 232 grams [70% yield] and purity was 96%.

### EXAMPLE 7

Identical to Example 6, but the reaction temperature was in the range of -20°C to -14°C. The yield was 188 grams [57%]. The major impurity was the ester amounting to 38%.

### EXAMPLE 8

This experiment was carried out in a 20-gallon kettle under conditions described in Example 4. The following quantities of chemicals and solvents were used.

| | | |
|---|---|---|
| C6F5-OK | 33.31 moles | = 7405 grams |
| HPPO [15% excess] | 38.3 moles | = 6357 grams |
| Tetraglyme | | = 40 liters |
| Dichloromethane | | = 4 liters |

After the reaction with HFPO the crude product was worked up as follows. Dichloromethane from the crude product was distilled in a large [22 liter] rotary evaporator at a bath temperature of 45°C and a vacuum of 200 mm Hg with continuous feed of the crude product into the evaporation flask at the same rate of distillation. When the pot could no longer hold additional quantities [about 12 liters], the vacuum was reduced to 100 mm Hg and maintained until no more solvent was collecting in the receiver. The flask was emptied to a storage vessel and a second batch was subjected to the same routine. When all the crude product had been subjected to the above operation, it was transferred in batches to a 12-liter distillation still and the 1:1-adduct was distilled at a vacuum of 30 mm Hg. The bulk of product distilled weighed 7145 grams [65%]. It had a purity of 95% with 3% of 2:1-adduct.

### EXAMPLE 9

This was carried out in a 20-gallon [76 liter] kettle. Adiponitrile [17 liters] and KF [580 grams; 10 moles] were transferred to the kettle under a nitrogen atmosphere and stirred for 20 minutes. Tetraglyme [1.6 liters] and the distilled 1:1-adduct [100 moles; 33 kilograms] were then transferred to the kettle and the contents were heated by circulating warm water [40°C] through the jacket of the kettle while maintaining stirring. When the internal temperature reached 35°C HFPO was fed in at such a rate to maintain the internal temperature in the range of 35°C to 41 °C. The reaction was rapid as very little reflux was seen in the dry-ice condenser. When the addition of HFPO was completed, it was stirred for an additional 30 minutes. Stirring was stopped and after 15 minutes, the lower [mostly 2:1-adduct with small quantities of 1:1-adduct] layer was removed. GC analysis of a sample of this showed no solvent. It weighted 42.67 kg. It was transferred to a still and distilled in vacuum. Initially, the vacuum was set at 30 mm Hg and the unreacted 1:1-adduct was distilled until the temperature reached 85°C. When no more distillation was seen as indicated by a drop in the head temperature below 40°C the vacuum was increased to a range of 6-10 mm Hg and the 2:1-adduct free of the 1:1-adduct was collected at a boiling range of 68°C to 78°C. The distilled 2:1-adduct weighed 39.6 kg [yield is 80% based on the charged 1:1-adduct].

## Claims

1. A process comprising, contacting, in a solvent mixture consisting essentially of tetraglyme and 1,2-dimethoxyethane or methylene chloride or a mixture thereof, and at a temperature of about -60°C to about 0°C, an alkali metal salt of pentafluorophenol and hexafluoropropylene oxide to produce a compound of the formula C₆F₅OCF(CF₃)CF₂OM (I), wherein M is said alkali metal;
provided that the total molar amount of said hexafluoropropylene oxide added to the process is about 1.0 to about 1.25 times the molar amount of said alkali metal salt of pentafluorophenol present in said process.

2. The process as recited in Claim 1 wherein said alkali metal is sodium or potassium.

3. The process as recited in Claim 1 wherein said alkali metal is potassium.

4. The process as recited in Claim 3 wherein said tetraglyme is about 80 to about 95 volume percent of said solvent mixture.

5. The process as recited in Claim 3 wherein said temperature is about -30°C to about -15°C.

6. The process as recited in Claim 3 wherein the total molar amount of said hexafluoropropylene oxide added to the process is about 1.0 to about 1.15 times the molar amount of said alkali metal salt of pentafluorophenol present in said process.

7. The process as recited in Claim 6 wherein said tetraglyme is about 80 to about 95 volume percent of said solvent mixture, and said temperature is about -30°C to about -15°C.

8. A process for the production of a pentafluorophenoxy substituted perfluoroether, comprising, contacting, at a temperature of about +10°C to about +80°C, in a solvent mixture consisting essentially of adiponitrile and tetraglyme, a compound of the formula C₆F₅OCF(CF₃)COF (III), with a catalytically effective amount of potassium fluoride and hexafluoropropylene oxide to produce a compound of the formula C₆F₅OCF(CF₃)CF₂OCF(CF₃)COF (II).

9. The process as recited in Claim 8 wherein said solvent mixture is about 75 to about 95 volume percent adiponitrile.

10. The process as recited in Claim 8 wherein a molar ratio of total HFPO to (II) is about 1.0 to about 1.2.

11. The process as recited in Claim 8 wherein said potassium fluoride is present in an amount of 2 to 10 mole percent of (III).

12. The process as recited in Claim 8 wherein said temperature is about +30°C to about +50°C.

13. The process as recited in Claim 12 wherein said solvent mixture is about 75 to about 95 volume percent adiponitrile, a molar ratio of total HFPO to (II) is about 1.1 to about 1.2, and said potassium fluoride is present in an amount of 2 to 10 mole percent of (II).

14. A process for the production of a pentafluorophenoxy substituted perfluoroether, comprising,
(a) contacting, in a solvent mixture consisting essentially of tetraglyme and 1,2-dimethoxyethane or methylene chloride or a mixture thereof, and at a temperature of about -60°C to about 0°C, an alkali metal salt of pentafluorophenol and hexafluoropropylene oxide to produce a compound of the formula C₆F₅OCF(CF₃)CF₂OM (I), wherein M is said alkali metal;
provided that the total molar amount of said hexafluoropropylene oxide added to the process is no greater than 1.25 time the molar amount of said alkali metal salt of pentafluorophenol present in said process to produce (I); and
(b) heating (I) to produce a compound of the formula C₆F₅OCF(CF₃)COF (III);
(c) contacting, at a temperature of about +10°C to about +80°C, in a solvent mixture consisting essentially of adiponitrile and tetraglyme, with a catalytic amount of potassium fluoride and hexafluoropropylene oxide to produce a compound of the formula C₆F₅OCF(CF₃)CF₂OCF(CF₃)COF (II).

15. The process as recited in Claim 14 wherein (b) is carried out at about +20°C to about +150°C.

16. The process as recited in Claim 14 wherein (b) is carried out at about +25°C to about +100°C.

17. A process for the production of a pentafluorophenoxy substituted perfluoroether, comprising:
(a) contacting, in a solvent mixture consisting essentially of tetraglyme and 1,2-dimethoxyethane or methylene chloride or a mixture thereof, and at a temperature of about -60°C to about 0°C, an alkali metal salt of pentafluorophenol and hexafluoropropylene oxide to produce a compound of the formula C₆F₅OCF(CF₃)CF₂OM (I), wherein M is said alkali metal;
provided that the total molar amount of said hexafluoropropylene oxide added to the process is no greater than 1.25 time the molar amount of said alkali metal salt of pentafluorophenol present in said process to produce (I);
(b) adding a completely perfluorinated compound which is a liquid under the process conditions; and
(c) then heating to about 25°C to about 50°C while adding approximately at least one equivalent of HFPO to produce a compound of the formula C₆F₅O[CF(CF₃)CF₂O]ₙCF(CF₃)COF (V), wherein n is an integer of 1 to 5.

18. The process as recited in Claim 17 wherein about one-half to about an equal volume compared to said solvent mixture of said perfluorinated compound is added.

19. The process as recited in Claim 17 wherein in (c) about one equivalent of said hexafluoropropylene oxide is added.

## Patentansprüche

1. Verfahren, umfassend Inkontaktbringen in einem Lösungsmittelgemisch, bestehend im wesentlichen aus Tetraglym und 1,2-Dimethoxyethan oder Methylenchlorid oder einem Gemisch davon, und bei einer Temperatur von etwa -60°C bis etwa 0°C von einem Alkalimetallsalz von Pentafluorphenol und Hexafluorpropylenoxid, um eine Verbindung der Formel C₆F₅OCF(CF₃)CF₂OM (I), wobei M das Alkalimetall ist, herzustellen;
mit der Maßgabe, daß die Gesamtmolmenge des Hexafluorpropylenoxids, hinzugegeben zu dem Verfahren, das etwa 1,0- bis etwa 1,25-fache der Molmenge des Alkalimetallsalzes von Pentafluorphenol, vorhanden in dem Verfahren, beträgt.

2. Verfahren nach Anspruch 1, wobei das Alkalimetall Natrium oder Kalium ist.

3. Verfahren nach Anspruch 1, wobei das Alkalimetall Kalium ist.

4. Verfahren nach Anspruch 3, wobei das Tetraglym etwa 80 bis etwa 95 Volumenprozent des Lösungsmittelgemisches ausmacht.

5. Verfahren nach Anspruch 3, wobei die Temperatur etwa -30°C bis etwa -15°C beträgt.

6. Verfahren nach Anspruch 3, wobei die Gesamtmolmenge des Hexafluorpropylenoxids, hinzugegeben zu dem Verfahren, das etwa 1,0- bis etwa 1,15-fache der Molmenge des Alkalimetallsalzes von Pentafluorphenol, vorhanden in dem Verfahren, beträgt.

7. Verfahren nach Anspruch 6, wobei das Tetraglym etwa 80 bis etwa 95 Volumenprozent des Lösungsmittelgemisches ausmacht und die Temperatur etwa -30°C bis etwa -15°C beträgt.

8. Verfahren für die Herstellung eines Pentafluorphenoxy-substituierten Perfluorethers, umfassend Inkontaktbringen bei einer Temperatur von etwa +10°C bis etwa +80°C in einem Lösungsmittelgemisch, bestehend im wesentlichen aus Adiponitril und Tetraglym, von einer Verbindung der Formel C₆F₅OCF(CF₃)COF (III) mit einer katalytisch wirksamen Menge von Kaliumfluorid und Hexafluorpropylenoxid, um eine Verbindung der Formel C₆F₅OCF(CF₃)CF₂OCF(CF₃)COF (II) herzustellen.

9. Verfahren nach Anspruch 8, wobei das Lösungsmittelgemisch zu etwa 75 bis etwa 95 Volumenprozent aus Adiponitril besteht.

10. Verfahren nach Anspruch 8, wobei ein Molverhältnis von Gesamt-HFPO zu (II) etwa 1,0 bis etwa 1,2 beträgt.

11. Verfahren nach Anspruch 8, wobei das Kaliumfluorid in einem Anteil von 2 bis 10 Molprozent von (III) vorhanden ist.

12. Verfahren nach Anspruch 8, wobei die Temperatur etwa +30°C bis etwa +50°C beträgt.

13. Verfahren nach Anspruch 12, wobei das Lösungsmittelgemisch zu etwa 75 bis etwa 95 Volumenprozent aus Adiponitril besteht, ein Molverhältnis von Gesamt-HFPO zu (II) etwa 1,1 bis etwa 1,2 beträgt und das Kaliumfluorid in einem Anteil von 2 bis 10 Molprozent von (II) vorhanden ist.

14. Verfahren für die Herstellung eines Pentafluorphenoxy-substituierten Perfluorethers, umfassend
(a) Inkontaktbringen in einem Lösungsmittelgemisch, bestehend im wesentlichen aus Tetraglym und 1,2-Dimethoxyethan oder Methylenchlorid oder einem Gemisch davon, und bei einer Temperatur von etwa -60°C bis etwa 0°C von einem Alkalimetallsalz von Pentafluorphenol und Hexafluorpropylenoxid, um eine Verbindung der Formel C₆F₅OCF(CF₃)CF₂OM (I), wobei M das Alkalimetall ist, herzustellen;
mit der Maßgabe, daß die Gesamtmolmenge des Hexafluorpropylenoxids, hinzugegeben zu dem Verfahren, nicht größer als das 1,25-fache der Molmenge des Alkalimetallsalzes von Pentafluorphenol, vorhanden in dem Verfahren, um (I) herzustellen, ist; und
(b) Erhitzen von (I), um eine Verbindung der Formel C₆F₅OCF(CF₃)COF (III) herzustellen;
(c) Inkontaktbringen bei einer Temperatur von etwa +10°C bis etwa +80°C in einem Lösungsmittelgemisch, bestehend im wesentlichen aus Adiponitril und Tetraglym, mit einer katalytischen Menge von Kaliumfluorid und Hexafluorpropylenoxid, um eine Verbindung der Formel C₆F₅OCF(CF₃)CF₂OCF(CF₃)COF (II) herzustellen.

15. Verfahren nach Anspruch 14, wobei (b) bei etwa +20°C bis etwa +150°C ausgeführt wird.

16. Verfahren nach Anspruch 14, wobei (b) bei etwa +25°C bis etwa +100°C ausgeführt wird.

17. Verfahren für die Herstellung eines Pentafluorphenoxy-substituierten Perfluorethers, umfassend:
(a) Inkontaktbringen in einem Lösungsmittelgemisch, bestehend im wesentlichen aus Tetraglym und 1,2-Dimethoxyethan oder Methylenchlorid oder einem Gemisch davon, und bei einer Temperatur von etwa -60°C bis etwa 0°C von einem Alkalimetallsalz von Pentafluorphenol und Hexafluorpropylenoxid, um eine Verbindung der Formel C₆F₅OCF(CF₃)CF₂OM (I), wobei M das Alkalimetall ist, herzustellen;
mit der Maßgabe, daß die Gesamtmolmenge des Hexafluorpropylenoxids, hinzugegeben zu dem Verfahren, nicht größer als das 1,25-fache der Molmenge des Alkalimetallsalzes von Pentafluorphenol, vorhanden in dem Verfahren, um (I) herzustellen, ist;
(b) Hinzugeben einer vollständig perfluorierten Verbindung, welche unter den Verfahrensbedingungen eine Flüssigkeit ist; und
(c) dann Erhitzen auf etwa 25°C bis etwa 50°C, während ungefähr mindestens ein Äquivalent von HFPO hinzugegeben wird, um eine Verbindung der Formel C₆F₅O[CF(CF₃)CF₂O]ₙCF(CF₃)COF (V), wobei n eine ganze Zahl von 1 bis 5 ist, herzustellen.

18. Verfahren nach Anspruch 17, wobei im Vergleich zu dem Lösungsmittelgemisch etwa eine Hälfte bis etwa ein gleiches Volumen der perfluorierten Verbindung hinzugegeben wird.

19. Verfahren nach Anspruch 17, wobei in (c) etwa ein Äquivalent des Hexafluorpropylenoxids hinzugegeben wird.

## Revendications

1. Procédé comprenant la mise en contact, dans un mélange de solvants constitué essentiellement de tétraglyme et de 1,2-diméthoxyéthane ou de chlorure de méthylène ou d'un mélange de ceux-ci, et à une température d'environ -60°C à environ 0°C, d'un sel de métal alcalin de pentafluorophénol et d'hexafluoropropylène pour produire un composé de formule C₆F₅OCF(CF₃)CF₂OM (I) dans laquelle M est ledit métal alcalin;
à condition que la quantité molaire totale dudit oxyde d'hexafluoropropylène ajoutée au procédé soit d'environ 1,0 à environ 1,25 fois la quantité molaire dudit sel de métal alcalin de pentafluorophénol présente dans ledit procédé.

2. Procédé selon la revendication 1, dans lequel ledit métal alcalin est le sodium ou le potassium.

3. Procédé selon la revendication 1, dans lequel ledit métal alcalin est le potassium.

4. Procédé selon la revendication 3, dans lequel ledit tétraglyme représente environ 80 à environ 95 pour cent en volume dudit mélange de solvants.

5. Procédé selon la revendication 3, dans lequel ladite température est d'environ -30°C à environ -15°C.

6. Procédé selon la revendication 3, dans lequel ladite quantité molaire totale dudit oxyde d'hexafluoropropylène ajouté au procédé est d'environ 1,0 à environ 1,15 fois la quantité molaire dudit sel de métal alcalin de pentafluorophénol présent dans ledit procédé.

7. Procédé selon la revendication 6, dans lequel ledit tétraglyme représente d'environ 80 à environ 95 pour cent en volume dudit mélange de solvants, et ladite température est d'environ -30°C à environ -15°C.

8. Procédé pour la production d'un perfluoroéther pentafluorophénoxy-substitué comprenant la mise en contact, à une température d'environ +10°C à environ +80°C, dans un mélange de solvants constitué essentiellement d'adiponitrile et de tétraglyme, d'un composé de formule C₆F₅OCF(CF₃)COF (III) avec une quantité catalytique de fluorure de potassium et d'oxyde d'hexafluoropropylène pour produire un composé de formule C₆F₅OCF(CF₃)CF₂OCF(CF₃)COF (II).

9. Procédé selon la revendication 8, dans lequel ledit mélange de solvants est d'environ 75 à environ 95 pour cent en volume d'adiponitrile.

10. Procédé selon la revendication 8, dans lequel un rapport molaire d'HFPO total à (II) est d'environ 1,0 à environ 1,2.

11. Procédé selon la revendication 8, dans lequel ledit fluorure de potassium est présent dans une quantité de 2 à 10 pour cent en mole de (III).

12. Procédé selon la revendication 8, dans lequel ladite température est d'environ +30°C à environ +50°C.

13. Procédé selon la revendication 12, dans lequel ledit mélange de solvants est d'environ 75 à environ 95 pour cent en volume d'adiponitrile, un rapport molaire d'HFPO total à (II) est d'environ 1,1 à environ 1,2, et ledit fluorure de potassium est présent dans une quantité de 2 à 10 pour cent en mole de (II).

14. Procédé pour la production d'un perfluoroéther pentafluorophénoxy-substitué comprenant
a) la mise en contact, dans un mélange de solvants constitué essentiellement de tétraglyme et de 1,2-diméthoxyéthane ou de chlorure de méthylène ou d'un mélange de ceux-ci, et à une température d'environ -60°C à environ 0°C, d'un sel de métal alcalin de pentafluorophénol et d'oxyde d'hexafluoropropylène pour produire un composé de formule C₆F₅OCF(CF₃)CF₂OM (I), dans laquelle M est ledit métal alcalin;
à condition que la quantité molaire totale dudit oxyde d'hexafluoropropylène ajouté au procédé ne soit pas supérieure à 1,25 fois la quantité molaire dudit sel de métal alcalin de pentafluorophénol présente dans ledit procédé pour produire (I);
b) le chauffage de (I) pour produire un composé de formule C₆F₅OCF(CF₃)COF (III); et
c) la mise en contact, à une température d'environ +10°C à environ +80°C, dans un mélange de solvants constitué essentiellement d'adiponitrile et de tétraglyme, avec une quantité catalytique de fluorure de potassium et d'oxyde d'hexafluoropropylène pour produire un composé de formule C₆F₅OCF(CF₃)CF₂OCF(CF₃)COF (II).

15. Procédé selon la revendication 14, dans lequel (b) est effectué à d'environ +20°C à environ +150°C.

16. Procédé selon la revendication 14, dans lequel (b) est effectué à d'environ +25°C à environ +100°C.

17. Procédé pour la production d'un perfluoroéther pentafluorophénoxy-substitué, comprenant :
a) la mise en contact, dans un mélange de solvants constitué essentiellement de tétraglyme et de 1,2-diméthoxyéthane ou de chlorure de méthylène ou d'un mélange de ceux-ci, et à une température d'environ -60°C à environ 0°C, d'un sel de métal alcalin de pentafluorophénol et d'oxyde d'hexafluoropropylène pour produire un composé de formule C₆F₅OCF(CF₃)CF₂OM (I), dans laquelle M est ledit métal alcalin;
à condition que la quantité molaire totale dudit oxyde d'hexafluoropropylène ajouté au procédé ne soit pas supérieur à 1,25 fois la quantité molaire dudit sel de métal alcalin de pentafluorophénol présente dans ledit procédé pour produire (I); et
b) l'addition d'un composé complètement perfluoré qui est liquide sous les conditions du procédé; et
c) ensuite le chauffage à d'environ 25°C à environ 50°C tout en ajoutant approximativement au moins un équivalent d'HFPO pour produire un composé de formule C₆H₅O[CF(CF₃)CF₂O]ₙCF(CF3)COF (V) dans laquelle n est un entier de 1 à 5.

18. Procédé selon la revendication 17, dans lequel on ajoute d'environ la moitié à environ un volume égal, comparé audit mélange de solvants, dudit composé perfluoré.

19. Procédé selon la revendication 17, dans lequel on ajoute en (c) environ un équivalent dudit oxyde d'hexafluoropropylène.
